(19) Europäisches Patentamt
European Patent Office
Office européen des brevets

(11) **EP 3 126 009 B1**

(12) **EUROPEAN PATENT SPECIFICATION**

(45) Date of publication and mention
of the grant of the patent:
**04.07.2018 Bulletin 2018/27**

(51) Int Cl.:
***A61Q 9/02*** *(2006.01)*     ***A61K 8/86*** *(2006.01)*
***B26B 21/44*** *(2006.01)*

(21) Application number: **15714753.9**

(22) Date of filing: **26.03.2015**

(86) International application number:
**PCT/US2015/022683**

(87) International publication number:
**WO 2015/153267 (08.10.2015 Gazette 2015/40)**

(54) **LUBRICATING SKIN ENGAGING MEMBER FOR RAZOR CARTRIDGES**

SCHMIERENDES HAUTKONTAKTELEMENT FÜR RASIERKLINGEN

ÉLÉMENT DE LUBRIFICATION ENTRANT EN CONTACT AVEC LA PEAU POUR CARTOUCHES DE RASOIR

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **03.04.2014 US 201461974658 P**

(43) Date of publication of application:
**08.02.2017 Bulletin 2017/06**

(73) Proprietor: **The Gillette Company LLC**
**Boston, MA 02127 (US)**

(72) Inventors:
• **STEPHENS, Alison, Fiona**
**Egham**
**Surrey TW20 9NW (GB)**
• **KEELING, Emma, Louise**
**Reading**
**Berkshire RG2 0QE (GB)**
• **WARRICK, Paul, Leslie**
**Reading**
**Berkshire RG2 0QE (GB)**
• **BRADFORD, Valerie, Jean**
**Boston, Massachusetts 02127 (US)**

(74) Representative: **Kohol, Sonia**
**N.V. Procter & Gamble**
**Services Company S.A.**
**Temselaan 100**
**1853 Strombeek-Bever (BE)**

(56) References cited:
**WO-A1-2014/176391     WO-A2-2012/051377**
**US-A1- 2012 030 945**

**Description**

FIELD OF THE INVENTION

The present invention concerns skin engaging members, for use on razor cartridges to provide improved lubrication.

BACKGROUND OF THE INVENTION

The use of shaving aids on razor blades to provide lubrication benefits during the shave is known. *See e.g.*, U.S. Patents 7,121,754; 6,298,558; 5,711,076; 5,134,775; 6,301,785, 2009/0223057 and 2006/0225285. Such shaving aids typically comprise a water-insoluble matrix material to provide structural integrity and a water-soluble polymer such as polyethylene oxide (polyox) in order to provide the lubrication during the shave once the water-soluble polymer enters solution with water present during shaving. Since the introduction of polyox as a shaving lubricant, however little development has been made in the field, even though polyethylene oxide polymers are not without limitations. For example, the use of polyethylene oxide polymers with a low molecular weight provides limited lubrication, and while improved lubrication may be seen when using polyethylene oxide polymer with higher molecular weights, this negatively impacts other aspects of the aqueous solution typically formed in-use. For example, the resultant viscosity in aqueous solution may also increase, leading to negatively perceived attributes, for example concerning the feeling of the shave for the user, particularly in respect of the lubricant. The prior art also describes the use of combinations of high and low molecular weight polyethylene oxide in order to balance these performance attributes. Nevertheless such combinations are also limited in their ability to improve performance and or suffer from other negative performance attributes.
More recently, however, some shaving aids in the form of skin engaging members have been produced that comprise a tray or container in which a shaving aid such as polyethylene oxide polymers is contained. *See e.g.*, U.S. 2011/0099815, WO2011/047221, WO2011/047222, WO2011/049892 and WO2011/050130.
The perceived performance of the lubricant is however not entirely based upon the chemistry itself but is also dependent upon the relationship and orientation of the tray or container containing the lubricant, to the razor cartridge itself and the skin contacting surface thereof. US 2012/0030945 discloses a shaving aid retention member for a lubricant in which the position of the front wall is below the shaving plane in order to reduce discomfort and skin bulge.
It has further been identified that the processing techniques used in the manufacture of such skin engaging members such as extrusion, may also impact the lubricating performance.
Consequently, there is still a need to further improve the performance of the lubricant without the negatives described and while ensuring that this delivery to the skin surface is maintained throughout the cycle of use of the razor cartridge.

SUMMARY OF THE INVENTION

According to a first aspect, a razor cartridge is provided comprising a housing comprising

a) two or more blades forming a blade shaving plane (P) and
b) at least one skin engaging member, said skin engaging member comprising:

i) a container having a base and at least one wall extending substantially vertically to a maximum height (h) and preferably perpendicular to said blade shaving plane (P) and a skin contacting surface, preferably having a dispensing orifice, and
ii) a lubricating material or mixture thereof releasably and substantially contained within said container; said lubricating material comprising from 50% to 99% by weight of a polyethylene oxide polymer or mixture thereof,

wherein said polyethylene oxide polymer has an average molecular weight of from 0.5 million to 10 million and wherein said height (h) of said wall of said container is from 0.1mm above said blade plane (P) to 0.6 mm below said blade plane (P) and are selected such that the relationship between said molecular weight of said polyethylene oxide polymer and said maximum height of said wall is represented by the area below the line according to the following formula:

$$\text{polyethylene oxide polymer molecular weight (million)} = 10 * h \text{ (mm)} + 7$$

BRIEF DESCRIPTION OF THE DRAWINGS

FIG. 1 is a side view of a hair removal device in accordance with the present invention.
FIGs. 2 to 6 are cross sectional side views of additional razor cartridges in accordance with the present invention.

DETAILED DESCRIPTION OF THE INVENTION

According to the invention, a hair removal device (e.g. a razor) is provided, which comprises a razor cartridge as described herein below, and preferably a handle (or grip portion) permanently or removably attached to the cartridge. The hair removal device can be manual or power driven and can be used for wet and/or dry applications. The razor cartridge may be replaceable and/or pivotally connected to the handle (e.g. via a cartridge connecting structure) and in turn or independently (e.g. permanently fixed) to a handle. In some embodiments, the cartridge connecting structure includes at least one arm to releasably engage the cartridge.

The razor cartridge comprises at least one skin engaging member which typically enables the loading of various lubricating materials onto a razor cartridge for delivery to the skin during the hair removal process. The details of the skin engaging member and its location on a razor cartridge according to the invention will be disclosed herein, with figures showing exemplary embodiments which can include various elements of the present invention. Those of skill in the art will understand that various combinations of elements described in the specification and disclosed in the figures can be used in accordance with the present invention.

The skin-engaging member (also termed a shaving aid member or skin-engaging shaving aid member) comprises a lubricant, or lubricating material, as well as mixtures/combinations thereof, as will be described below. The lubricating material is releasably and substantially contained within the container.

I. Polyethylene oxide

The lubricating material comprises a polyethylene oxide polymer or mixtures thereof. The preferred polyethylene oxides are generally known as POLYOX (available from Union Dow Chemical Company) as WSR-N-12K nd WSR N-60K for example or ALKOX (available from Meisei Chemical Works, Kyoto, Japan). The polyethylene oxides has an average molecular weight of from about 0.5 million to about 10 million, preferably from about 1 million to about 6, million, more preferably from about 1 million to about 5 million, even more preferably from about 1 million to about 4 million and most preferably from about 1 million to about 2 million. It has been surprisingly found that the combination of polyethylene oxide at selected average molecular weights in combination with the location of the container wall height with respect to the blade plane, as decribed hereinafter, provides improved lubrication.

The polyethylene oxide will preferably comprise at least about 50%, more preferably at least about 60%, and up to about 99%, (or up to about 90%) by weight of the lubricating material. For example, the polyethylene oxide polymer may be present at an amount of at least about 50%, preferably from about 50% to about 99.9%, more preferably from about 60% to about 95% (e.g. from about 90% to about 95%) and even more preferably from about 70% to about 90% by weight of the lubricating material.

Not all of the polyoxythylene oxide polymer needs to meet the average molecular weight requirement, for example a blend of two or more grades of polyethylene oxide could be used wherein at least one, but less than all, of the grades meets the average molecular weight attribute, but the total amount of polyethylene oxide is within one of the ranges above. Alternatively, the average molecular weight for the entirety of the polyethylene oxide may fall within the desired average molecular weight range as well as the total amount of the polyethylene oxide being according to one or more of the ranges above.

II. Water soluble polymers

The lubricating material may further comprise additional water soluble polymers. Examples of suitable water soluble polymers include polyvinyl pyrrolidone, polyacrylamide, polyhydroxymethacrylate, polyvinyl imidazoline, polyethylene glycol, polyvinyl alcohol, polyhydroxyethymethacrylate, silicone polymers, and mixtures thereof. In some embodiments, said water soluble polymer is selected from the group consisting of polyethylene glycol, and mixtures thereof. Such water soluble polymers may be used at levels of from 1% to 50%, preferably from 1% to 20% by weight of the lubricating material.

III. Copolymer of Polyethylene Oxide and Polypropylene Oxide

The lubricating material may further comprise a copolymer of polyethylene oxide (PEO) and polypropylene oxide (PPO). The PEO/PPO copolymer may have an average molecular weight of at least 5,000, preferably in the range of from 10,000 to 20,000, more preferably from 11,000 to 15,000, even more preferably from 12,000 to 13,000 and even more preferably still from 12,250 to 12,750. Without wishing to be bound by theory, the inclusion of a PEO/PPO copolymer of sufficient molecular weight is thought to further improve the lubrication properties of the skin engaging member in aqueous conditions, especially in combination with a further water soluble polymer (particularly polyethylene oxide), and thus prevent an undesirable feeling in use.

The PEO/PPO copolymer may advantageously be a block copolymer, for example a di-block, tri-block, multi-block, radial-block or random-block copolymer. Preferably, the PEO/PPO copolymer is a tri-block copolymer, more preferably a tri-block copolymer having the sequence: PEO-PPO-PEO, the later commercially available under tradenames such as Pluracare from BASF and Pluronic from Sigma-Aldrich.

The PEO/PPO copolymer may have a weight ratio of PEO to PPO (i.e. of ethylene oxide repeat units to propylene oxide repeat units), of from 1000:1 to 1:1000 or from 100:1 to 1:100. Advantageously, the weight ratio is selected to improve the solubility properties of the PEO/PPO copolymer in a system comprising a water-soluble polymer (especially polyethylene oxide) and water, and so may be from 10:1 to 1:10, preferably from 1:1 to 1:7 (or any ratio in which the weight of PPO is greater than or equal to the weight of PEO), more preferably from 1:2 to 1:5, even more preferably from 1:2.5 to 1:4 and even more preferably still from 1:2.5 to 1:3. The PEO/PPO copolymer may have an HLB of from 0 to 50, advantageously from 1 to 30, preferably from 5-25, more preferably from 10-25, even more preferably from 17-24 and even more preferably still from 18-23.

The PEO/PPO copolymer is typically present at an amount of from 0.01% to 50%, preferably from 0.01% to 50%, more preferably from 2% to 40%, even more preferably from 3% to 25%, even more preferably still from 4% to 20% and most preferably from 5% to 10% by weight of the lubricating material or by weight of the skin engaging member.

IV. Water Insoluble Material

The lubricating material may also further comprise a water-insoluble material. Suitable materials include a hydrophobic binder. Such a component may enhance the life of the lubricating material by reducing its tendency to be mechanically eroded. Advantageously, the hydrophobic binder is solid at standard temperature and pressure. Suitable hydrophobic binders include divalent metal cation stearate, preferably magnesium stearate, calcium stearate, zinc stearate, or mixtures thereof, more preferably magnesium separate; ethyl cellulose; polycaprolactone; polyethylene; polypropylene; polystyrene; butadiene-styrene copolymer (e.g. medium and high impact polystyrene); polyacetal; acrylonitrilebutadiene-styrene copolymer; ethylene vinyl acetate copolymer and blends such as polypropylene/polystyrene blend; and mixtures thereof. In the event that the lubricating material is cold-pressed, then it preferably comprises from 1 to 20% and more preferably from 5 to 15% hydrophobic binder, by weight of the lubricating material. In the event that the lubricating material is manufactured by an extrusion process or injection molding, as discussed below, then it preferably comprises from 10 to 50%, more preferably from 15 to 40% and yet more preferably 20 to 35% hydrophobic binder, by weight of the lubricating material.

V. Further Optional Ingredients

In some embodiments, the lubricating material may comprise any other ingredients commonly found in commercially available shaving aid members or skin engaging members, such as those used on razor cartridges by Gillette, Schick or BIC. Non-limiting examples of such skin engaging members include those disclosed in U.S. 6,301,785; 6,442,839; 6,298,558; 6,302,785; 2008/060201, and 2009/0223057.

The lubricating material may therefore contain other conventional shaving aid ingredients, such as low mol.wt. water-soluble release enhancing agents such as polyethylene glycol (MW<10,000, e.g., 1-10% by weight PEG-100), water-swellable release enhancing agents such as cross-linked polyacrylics (e.g., 2-7% by weight), colorants, skin feel / care actives, surfactants, soaps (including interrupted soaps), antioxidants, preservatives, emollients, lipids, oils, waxes, fats, cooling agents (especially non-volatile cooling agents), essential oils, beard softeners, astringents, medicinal agents, plasticizers, additional lubricants, depilatories/keratolytic materials, tackifiers, skin-soothing agents, fragrances, compatibilisers, anti-inflammatory agents, antipruritic/counterirritant materials etc and mixture thereof.

VI. Lubricating material

The lubricating material is preferably provided in the container in the form of a compressed powder. As used herein the term "compressed powder" refers to a particulated lubricating material which is subsequently compressed, preferably using cold compression techniques. Preferably the particle size is such that 90% of particles pass through a 20 mesh screen; i.e. 90% of particles are less than 841micron in diameter. The lubricating material is compressed into the container with a compression force of greater than 1KN. This may be achieved using any method and equipment known in the art such as a die press.

The lubricating material itself may have an average molecular weight of from about 0.5 million to 10 million preferably from about 1 million to 6 million, more preferably from about 1 million to 5 million, even more preferably from about 1 million to 4 million, and most preferably from about 1 million to 2 million. It has been surprisingly found that the combination of lubricating material at selected average molecular weights in combination with the location of the container for the lubricating material maximum wall height with respect to the blade plane, as described hereinafter, provides improved lubrication.

While not bound by theory it is believed that certain manufacturing processing techniques used to form the skin engaging member in particular the lubricating material may result in heat induced degradation affecting the molecular weight of the resultant lubricating material. In a preferred embodiment the lubricating material is cold compressed into the container.

VII. Skin Engaging Member:

The razor cartridge comprises at least one or two skin engaging member(s). The skin engaging member(s) comprises a container having a base and at least one side wall extending vertically preferably perpendicular from said base and a skin contacting surface. In a preferred embodiment said container comprises a base and at least 2 side walls, more preferably at least 4 side walls, preferably said walls completely enclosing the base. Typically, each pair of walls are substantially parallel and preferably one pair of walls is substantially parallel to the at least two blades. Alternatively, the base may be enclosed by a one piece single wall. The container may form any shape including substantially rectangular, or oval. The container typically has a front wall adjacent the blades and a rear wall, preferably substantially parallel thereto and furthest from said blades.

The container is preferably further provided with at least one dispensing orifice for dispensing the lubricating material onto the skin during use. For example the skin engaging member may comprise a container into which at least one orifice is formed. In one embodiment the container is provided with a top extending substantially perpendicular from the side wall(s). The container would in such an embodiment typically have a receiving region for receiving the lubricating material. The top may be substantially parallel to the base or it may be provide at an angle such that the distance of the top from the blade plane increases or decreases as the distance of the container from the blades increases. In one embodiment the height of the top of the container increases in distance from the blade plane as the container distance from the blades increases. In an alternative embodiment the height of the top of the container decreases in distance from the blade plane as the container distance from the blade increases.

The orifice may be of any shape and may, for example, have a cross sectional area of from about 0.00324 to about 1.613 cm$^2$. Small orifices can also be provided with a cross sectional area of from about 0.0324 to about 0.324 cm$^2$, or from about 0.0645 to about 0.16135 cm$^2$. Larger orifices can have cross sectional areas of from about 0.324 to about 1.613 cm$^2$, or from about 0.645 to about 1.29 cm$^2$. The container may comprise a single orifce or multiple orifices which may be large and or small. In one embodiment the container comprises at least two orifices. Combinations of small and large orifices can also be provided on the same skin engaging member, or on separate skin engaging members on the same cartridge, depending on the desired dispense rate and amount of exposure of the lubricating material to water. In one embodiment the top of the skin engaging member is provided with one preferably two orifices, more preferably two substantially identical orifices adjacent one another.

The skin engaging member will typically have a skin engaging surface which has a surface area, while the at least one orifice (i.e. the sum for all orifices if a plurality are present) has a cross sectional area such that the surface area and cross sectional area are in a ratio of from about 50:1 to about 1:1, or about 25:1 to about 2:1, or about 10:1 to about 3:1. An individual orifice may also have a greatest lateral distance, varying depending on the number of orifices used, for example from about 1% to about 99% of the greatest lateral distance of the hair removal cartridge or a portion of the skin-engaging member corresponding to the exposed length of any hair removal members, or from about 2% to about 95%, or from about 3% to about 90%, or from about 5% to about 80%, or from about 10% to about 75%, or from about 15% to about 50%. Examples of suitable carriers include the sheaths disclosed in U.S. Patent No. 6,298,558 or 7,581,318. The one or more orifices may be arranged so as to substantially or entirely cover an area or length of the skin-engaging member, or a portion of the skin-engaging member corresponding to the exposed length of any hair removal members, i.e. the length available for hair removal, e.g. not covered as clips. For example, a plurality of orifices may be arranged in an array that spans substantially or entirely the exposed length of any hair removal members, or of the greatest lateral distance of the hair removal cartridge, in order that lubricating material may be provided across the area being shaved. Alternatively, the one or more orifices may be arranged so as to cover an area or length of the skin-engaging member or a portion of the skin-engaging member corresponding to the exposed length of any hair removal members in accordance with any of the percentage ranges or values described for the individual orifices above.

In some embodiments, at least a portion of said skin engaging member and or container is not linear for example angled or curvilinear. Curvilinear as defined herein means that at least a portion is curved such that it does not form a straight line. Where at least two skin engaging members are provided, they can also be positioned relative to one another such that they do not form a straight line.

In some embodiments, the curved or angled nature of the skin engaging member is such that it forms at least a partial ring. A partial ring, as defined herein, means that the structure has at least two curved or angled sections which are concave to form an inner region. The partial ring can also include a curved or angled portion which is positioned convex to said inner region. One or more of said skin engaging members may also be positioned relative to one another to form a full ring. The ring can be formed by a single skin engaging member but two or more can be touching at or about their terminal ends, or even overlapping, to form such a ring.

The container of the skin engaging member, i.e. the physical structure of the skin engaging member in the absence of the lubricating material, can be formed of a variety of materials. The container may, preferably be for example, provided from a non-water soluble material such that it does not degrade or dissolve during normal use.

The container typically has sufficient mechanical strength and rigidity to provide adequate mechanical strength to the entire skin engaging member, both as initially produced and after a significant amount of lubricating material has leached out of the container. Alternatively or in addtion a further reinforcing member may also be utilized. In some embodiments, the container comprises a base and one or more side walls, forming a receiving region, or channel, onto or into which the lubricating material is placed. The carrier may also form one or more retaining members within said receiving region extending away from said base. The retaining member may also be elongated and stretch across at least about 20% to about 100%, or from about 35% to about 75%, or about 50%, of the length or width of the container. A plurality of retaining members may also be used, for example in a linear or non-linear alignment throughout the receiving region, and can be spaced equidistantly or not. The retaining member(s) may have a height of from about 0.05 cm to about 1 cm or from about 0.1 cm to about 0.5 cm. In other words, one or more (e.g. all) of the retaining members may have a height of from about 10% to about 100%, or from about 30% to about 60%, of the height of at least one of said side walls.

The side walls may or may not be the same height (as measured extending away from the base of the container). At least one of the side walls can have a height of about 0.1 cm to about 1 cm, preferably from about 0.2 cm to about 0.4 cm. The pair of side walls can be biased away from each other as the walls extend away from said base, or they can be biased towards each other. At least one wall extends vertically from the base and is preferably perpendicular to the blade plane (P). One or both ends of the container can be enclosed, e.g. as described in US 7,581,318. The term maximum height of at least one wall as used herein refers to the first front wall preferably substantially parallel to the at least two blades and closest thereto or it refers to the rear wall farthest from said at least two blades. In one embodiment the said at least one wall is closest to said at least two blades. In an alternative embodiment the at least one wall is farthest from said at least two walls. In one embodiment, the ratio of the height of the front wall to the rear wall is from 5:1 to 1:5, more preferably from 2:1 to 1:2 and more preferably the height of the front wall is greater than the rear wall. The walls have a thickness of from 0.1cm to 1.0cm, preferably from 0.3 to 0.5cm.

The container is a preferably filled with at least 60%, preferably at least 80%, more preferably 100% with said lubricating material. The container volume may also be over filled such that the container contains at least 105%, preferably at last 110% more preferably at least 120% of said lubricating material by volume.

The carrier may be made of a water-insoluble polymer, particularly a thermoplastic resin. Thermoplastic resins are those materials which can be extruded or molded into a shape and are resilient under normal environmental conditions such as contact with water, even up to normal household hot water temperatures (for example up to 125 °C); normal wear and tear by consumers during use; device assembly and shipping, etc. Thermoplastic resins suitable for use in the carrier include polystyrene, high impact polystyrene (polystyrene-butadiene), polypropylene, filled polypropylene, polyethylene, nylon ethylene vinyl acetate, and blends such as 70% nylon/30% polyethylene oxide, 60% polystyrene/40% polyethylene oxide butadiene styrene copolymer, polyacetal, acrylonitrile-butadiene styrene copolymer, and mixtures thereof. The preferred resins are high impact polystyrene, polystyrene, ethylene vinyl acetate (EVA), and mixtures thereof.

VIII. Razor Cartridge

According to the invention, a razor cartridge is provided, comprising a housing, at least two or more blades forming a blade shaving plane (P) and at least one skin engaging member as described hereinabove. The blade plane is defined by the horizontal plane extending from the location and orientation of the first and last blade in the housing. The skin engaging member, (e.g. via the container), may be affixed to the cartridge by any suitable means known in the art such as adhesive such as Loctite Super Bonder 499, mechanical locking mechanisms, thermal welds, co-molding with the cartridge housing or by a combination thereof.

Advantageously, in one embodiment, at least 30%, or the majority (i.e at least 50%), or least 75%, or up to 100% of the outer periphery of the at least two or more blades may be surrounded by one or more containers (for example one skin engaging member forward of the blades and one aft of the blades). A single container may be used to surround at least a portion of said two or more blades, or multiple containers and/or various compositions within the same containers may be used to surround at least a portion of said two or more blades. The "periphery" of the blades, as used herein, means the outer periphery of the structure upon which the hair removal member(s) are present for example the housing. The term surrounding as used herein does not necessarily require that at least 2 or more blades are enclosed by said container and the container may be positioned adjacent and parallel to said at least two or more blades.

The at least one skin engaging member may at least partially surround at least two sides of the two or more blades and in one embodiment may form a partial ring, or fully surround the entire periphery of the at least two blades. As explained above, the at least one skin engaging member can be formed of a single skin engaging member or can be two or more skin engaging members.

The razor cartridge comprises at least two, preferably 3 or more, more preferably 4 or more, most preferably 5 blades usually positioned between a first and second end. Said two or more elongated edges comprise a tip extending towards said first end and located with the housing of the razor cartridge. For example, U.S. 7,168,173 generally describes a Fusion® razor that is commercially available from The Gillette Company and which includes a razor cartridge with multiple blades. Additionally, the razor cartridge may include a guard as well as a skin engaging member. A variety of razor cartridges can be used in accordance with the present invention. Nonlimiting examples of suitable razor cartridges, with and without fins, guards, and/or shave aids, include those marketed by The Gillette Company under the Fusion®, Venus® product lines as well as those disclosed in U.S. 7,197,825; 6,449,849; 6,442,839; 6,301,785; 6,298,558; 6,161,288 and U.S. 2008/060201. Those of skill in the art will understand that the present skin engaging member can be used with any currently marketed system or disposable razor, including those having 2, 3, 4, 5 or more blades.

In some embodiments, said at least one skin engaging member is located on the portion of the cartridge that contacts skin during the shaving process, forward, between and/or aft of the at least two blades. A feature "forward" of the two or more blades, for example, is positioned so that the surface to be treated with by the shaving device encounters the feature before it encounters the hair removal members. A feature "aft" of a hair removal member is positioned so that the surface to be treated by the shaving device encounters the feature after it encounters the hair removal member. Where more than one skin engaging member is provided on the shaving device, they can be the same (identical) or different, in terms of physical shape/structure and/or chemical composition. In one preferred embodiment said skin engaging member is located aft the at least two blades.

The skin engaging member or each skin engaging member for embodiments having 2 or more skin engagement members is/are positioned on the housing such that the blade plane is independently above or below the maximum height of the at least one wall of the container of each skin engaging member. It has been found that the combination of specifc molecular weight of the polyethylene oxide and preferably the lubricating material and maximum wall height (h) independently of each container if multiple according to the formula herein results in an improvement of lubrication. Surprisingly it has been found that improved lubrication performance can be delivered by having the maximum height of at least one wall of the skin engaging member at a maximum level (h) above and below the blade plane when combined with specific molecular weight of polyethylene oxide of the lubricating material. The maximum height (h) of the wall of the container is from about 0.1mm above to about 0.6mm below, preferably from 0.00mm to 0.3mm below the blade plane and the molecular weight of said polyethylene oxide polymer is from about 0.5 million to about 10 million, preferably from about 1 million to about 6 million, more preferably from about 1 million to about 5 million, even more preferably from about 1 million to about 4 million, and most preferably from about 1 million to about 2 million. The relationship of the molecular weight of the polyethylene oxide polymer (million) and the height (mm) of the maximum height of the wall of each container is determined such that the relationship between said molecular weight of said polyethylene oxide polymer and said maximum height of said wall is represented by the area below the line according to the formula:

$$ML\ wt\ PO\ (million) = 10 * h\ (mm) + 7$$

and preferably according to the formula

$$ML\ wt\ PO\ (million) = 15 * h\ (mm) + 6.$$

While not bound by theory, polyethylene oxide copolymer with a higher molecular weight typically requires more load or force to be exerted in order to deliver the lubricating benefit due to the interaction with the aqueous environment. However such high loads are not consumer desirable. Therefore it is believed that the performance of a given polyethylene oxide polymer is improved by combining with a selected container height in order to provide both acceptable load and lubrication to the consumer.

In some embodiments, the cartridge comprises a guard comprising at least one elongated flexible protrusion to engage a user's skin. The at least one flexible protrusion may comprise flexible fins generally parallel to the one or more elongated edges (blades). Said at least one flexible protrusion may additionally or alternatively comprise flexible fins comprising at least one portion which is not generally parallel to said one or more elongated edges. Non-limiting examples of suitable guards include those used in current razor blades and include those disclosed in U.S. 7,607,230 and 7,024,776; (disclosing elastomeric/flexible fin bars); 2008/0034590 (disclosing curved guard fins); 2009/0049695A1 (disclosing an elastomeric guard having guard forming at least one passage extending between an upper surface and a lower surface). In some embodiments, said skin engaging member is positioned on the cartridge aft of the guard and forward of the hair removal members. In another embodiment, the skin engaging member is positioned on the cartridge forward of the guard. This embodiment can be particularly useful to deliver the lubricating material prior to contact with the guard.

The cartridge may further comprise an additional lubricating material (such as a known or commercially available shave aid or lubrication strip) to provide further lubrication to the hair removal experience. This additional lubricating material typically comprises a water insoluble polymer and a water soluble polymer. Such a shave aid or lubrication strip can be positioned in adjacent the skin engaging member, or forward, between or "aft" the at least two blades. In an alternative embodiment, the lubrastrip may be positioned to partial or completely encircle the razor cartridge housing. Non-limiting examples of known lubricating materials suitable for use herein include shave aids and lubrication strips as described in: U.S. 7,069,658; 6,944,952; 6,594,904; 6,302,785; 6,182,365; D424,745; 6,185,822; 6,298,558; 5,113,585, and 2009/0223057. The cartridge may also further comprise a cap.

IX. Figures

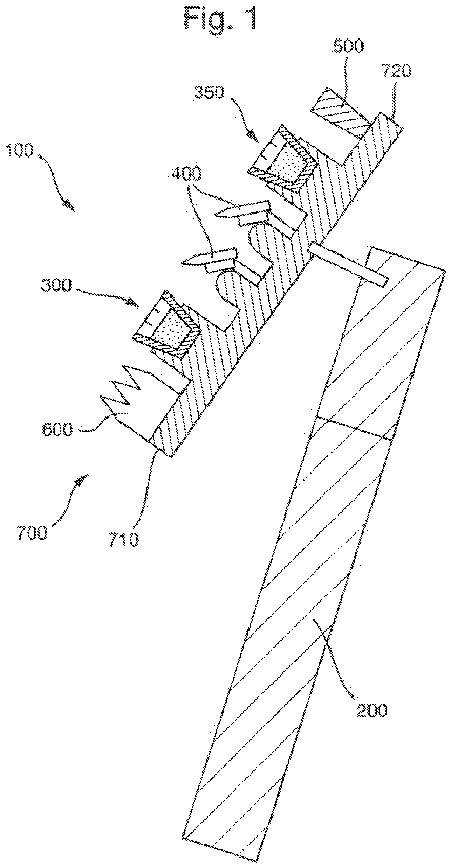

FIG. 1 is a side view of a hair removal device (100) in accordance with at least one embodiment of the present invention. The device comprises a razor cartridge (700) having a first end (710) and a second end (720), said cartridge being operably connected to a handle (200). In this example the hair removal cartridge includes two elongated edges (e.g. blades) (400), and a skin engaging member (300) positioned forward of said two elongated edges, and a skin engaging member (350) positioned aft of said two elongated edges. Further, a guard (600) is provided forward of said skin engaging member. An optional shave aid (500) is provided aft of said elongated edges. The skin engaging members (300 and 350) are below the blade plane.

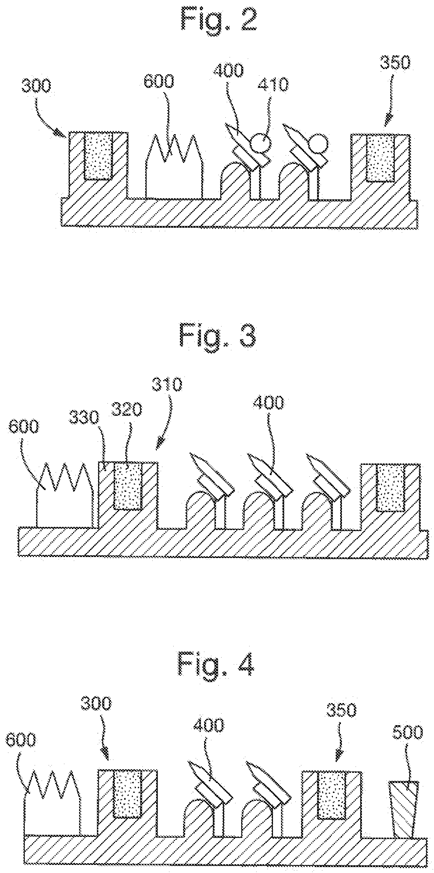

FIG. 2 is a cross sectional side view of another razor cartridge in accordance with at least one embodiment of the present invention. Two elongated edges (400) of razor blades are shown, having intrablade guards (410). In this example the first skin engaging member (300) is positioned forward of the elongated edges and forward of the guard, wherein the guard (600) is positioned between the elongated edges and the skin engaging member (300). Further shown here is a second skin engaging member (350), positioned aft of the elongated edges. The first and the second skin engaging members are preferably part of the same skin engaging member but they may also be different and separate structural elements.

FIG. 3 shows three elongated edges of blades provided on the hair removal cartridge. Also shown in this figure is the container (310) forming an orifice (320) to allow the lubricating material (330) to be dispensed during use onto skin. In this figure, the guard (600) is positioned forward of the first skin engaging member.

FIG. 4 shows the guard (600) positioned forward but not adjacent to the first skin engaging member (300) and a conventional solid polymeric shaving aid (500) positioned aft of the second skin engaging member (350).

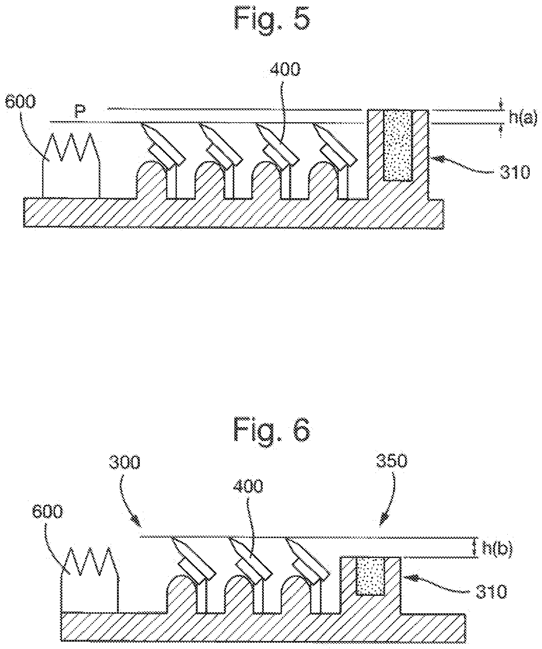

FIG. 5. shows four elongated edges of blades, a guard (600) positioned forward of the elongated edges and the skin engaging member (300) is aft of the elongated edges. The container (310) of the skin engaging member has a wall with a maximum height above (ha) the blade plane (P).

FIG. 6 shows three elongated edges of blades, a guard (600) positioned forward of the elongated edges and the skin engaging member (300) is aft of the elongated edges. The container (310) of the skin engaging member has a wall with a maximum height below (hb) the blade plane (P).

X. Methods of Making

The lubricating material can be manufactured in a variety of ways. Non-limiting examples of suitable methods include cold compression with or without ultrasonics or heat, extrusion and molding. Preferably, the lubricating material is provided in the form of a compressed powder. As used herein the term "compressed powder" refers to the lubricating material which is compressed from its original state to form a fused solid of interconnected particles. This solid will have substantially reduced porosity compared to the starting 'bulk' lubricating material. The lubricating material is formed into the compressed powder using known methods in the art. For example cold compression using a pressure of greater than 1KN, preferably from 2 to 5KN for a hold time of about 0.5 to 10 seconds, preferably about 0.5 to about 5 seconds, more preferably from about 0.5 seconds to about 2 seconds directly into a die. The compression may be carried out before or after insertion into the container.

The skin engaging member can be made in a variety of ways. The skin engaging member may also be made by producing the container (e.g. by injection molding), and then compressing the lubricating material into/onto the container, e.g. with a ram and with or without the application of heat, or ultrasonically tamping the lubricating material into the container, e.g. having poured it into the container in powdered form. The skin engaging member may be manufactured by co-extrusion (i.e. via a single extrusion die with two or more orifices so the extrudates merge and weld together as extruded) or two-colour molding/two-component molding (i.e. where the container or the lubricating material is first molded into a shape which can then be used to mold the other of the container or the lubricating material), or by extrusion or molding the container and lubricating material separately then assembling in a downstream step in the same process, or in a separate process/location. As explained above, the skin engaging member can be deformed to include a non-linear portion before or after the lubricating material is contacted with the container carrier.

XI. Methods of Hair Removal

The razor cartridge of the present invention may be used for hair removal (especially shaving), or in a method of hair removal (especially shaving), the method comprising the steps of providing a razor cartridge or hair removal device (especially a razor) according to the present invention in any form, and passing the same over a surface of the body. Optional additional steps may include wetting the surface, washing the surface, applying one of various commonly known shaving preparations to the surface, (the preceding options typically occurring before passing the hair removal/razor cartridge or hair removal device/razor over the surface), rinsing the surface (which could occur prior to and/or after passing the hair removal/razor cartridge or hair removal device/razor over the surface), drying the surface and applying one of various commonly known post-shave compositions to the surface (the last two steps typically occurring after passing the hair removal/razor cartridge or hair removal device/razor over the surface)

XII. Examples

Razor cartridges based upon commercially available Gillette ProGlide Power are prepared for testing with the following modifications:

The rear lubrastrip housing component of a commercially available pre-assembled ProGlide Power cartridge is machined away from the cartridge. A skin engaging member comprised of a rectangular shaped container of the following dimensions 33.8mm (1) 3.6mm (h) 3.2mm (w), with skin contacting surface having two centrally located dispensing orifices having dimensions of 14.2mm (1) and 1.2mm (w) separated by 0.5mm to form a total length of 28.9mm The container was produced by injection molding from a blend of polyphenylene oxide and polystyrene (available under the tradename "Noryl"). The skin engaging member is attached to the cartridge by adhering with a dichloromethane/trichloroethylene based adhesive. The lubricating materials in the table below were made by dry mixing the ingredients in the examples provided below and compression compacting 150mg into the skin engaging member container using a die press at 2.2KN for about 5 seconds.

| Example | Comparative | Inventive Example 1 | Inventive Example 2 | Inventive Example 3 |
|---|---|---|---|---|
| **Ingredient** | | | | |
| Polyethylene polymer (POLYOX from Union Dow Chemical Company) | 90.00 | 90.00 | 90.00 | 90.00 |
| MI wt (MM) | 5 | 1 | 2 | 2 |
| Pluronic F127(PEO-PPO Copolymer) | 10.00 | 10.00 | 10.00 | 10.00 |
| Height (h)mm above or below blade plane (P) | 0.4 (below) | 0.2 (below) | 0.4 (below) | 0.2 (below) |

| Example | Inventive Example 4 | Inventive Example 5 | Inventive Example 6 |
|---|---|---|---|
| **Ingredient** | | | |
| Polyethylene polymer (POLYOX from Union Dow Chemical Company) | 90.00 | 90.00 | 90.00 |
| MI wt (MM) | 2 | 4 | 5 |
| Pluronic F127(PEO-PPO Copolymer) | 10.00 | 10.00 | 10.00 |
| Height (h)mm above or below blade plane (P) | 0.00 | 0.2 (below) | 0.00 |

XIII. Consumer testing

94 UK based male wet shavers (current Gillette Fusion users excluding Fusion Power and Fusion ProGlide Power) aged between 18 and 54 who shave at least 3 times a week, were recruited for the testing panel. Each panellist was provided with 3 randomised products from the 7 products listed above. The panellists were requested to shave using the panellists own shaving preparation and after shave products using one test product each week. After each shave the panellist was asked to complete a questionnaire and an additional weekly questionnaire after using the test product for a week. The panellists were asked to rate the overall shave and glide performance using the following scale: Poor (1), Fair (2), Good (3), Very Good (4) to Excellent (5). The results of the testing are given below:

| Example | Comparative | 1 | 2 | 3 | 4 | 5 | 6 |
|---|---|---|---|---|---|---|---|
| Score | 3.15 | 3.44 | 3.43 | 3.34 | 3.77 | 3.33 | 3.67 |
| MI wt (MM) | 5 | 1 | 2 | 2 | 2 | 4 | 5 |
| Height (h)mm above or below blade plane (P) | 0.4 (below) | 0.2 (below) | 0.4 (below) | 0.2 (below) | 0.00 | 0.2 (below) | 0.00 |

From the data it can clearly be seen all of the inventive example provide a consumer noticeable lubrication benefit.

As used herein, molecular weights (mol.wt.s) are provided in unified atomic mass units, daltons, or g/mol.

It should be understood that every maximum numerical limitation given throughout this specification includes every lower numerical limitation, as if such lower numerical limitations were expressly written herein. Every minimum numerical limitation given throughout this specification includes every higher numerical limitation, as if such higher numerical limitations were expressly written herein. Every numerical range given throughout this specification includes every narrower numerical range that falls within such broader numerical range, as if such narrower numerical ranges were all expressly written herein.

All parts, ratios, and percentages herein, in the Specification, Examples, and Claims, are by weight and all numerical limits are used with the normal degree of accuracy afforded by the art, unless otherwise specified.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a document incorporated by reference, the meaning or definition assigned to that term in this document shall govern.

**Claims**

1. A razor cartridge is provided comprising a housing comprising

       a) two or more blades forming a blade shaving plane (P) and

b) at least one skin engaging member, said skin engaging member comprising:

i) a container having a base and at least one wall extending substantially vertically to a maximum height (h) and preferably perpendicular to said blade shaving plane (P) and a skin contacting surface preferably having a dispensing orifice, and

ii) a lubricating material or mixture thereof releasably and substantially contained within said container, said lubricating material comprising from 50% to 99% by weight of a polyethylene oxide polymer or mixture thereof, wherein said polyethylene oxide polymer has an average molecular weight of from 0.5 million to 10 million and wherein

said height (h) of said wall of said container is from 0.1mm above said blade plane (P) to 0.6 mm below said blade plane (P) and are selected such that the relationship between said molecular weight of said polyethylene oxide polymer and said maximum height of said wall is represented by the area below the line according to the following formula:

$$\text{molecular weight (million) polyethylene oxide polymer} = 10 * h\ (mm) + 7.$$

2. The razor cartridge according to claim 1, wherein said molecular weight of said polyethylene oxide polymer and said maximum height (h) of said at least one wall of said container are selected such that the relationship between said molecular weight of said polyethylene oxide polymer and said maximum height of said wall is represented by the area below the line according to the formula:

$$\text{Molecular weight (million) polyethylene oxide polymer} = 15 * h\ (mm) + 6.$$

3. The razor cartridge according to claim 1, wherein said height of said wall (h) of said container is from 0mm to 0.3mm below said blade plane (P).

4. The razor cartridge according to claim 1, wherein said polyethylene oxide polymer has an average molecular weight of from 1 million to 6 million, preferably from 1 million to 5 million.

5. The razor cartridge according to claim 1, wherein said polyethylene oxide polymer has a molecular weight of from 1 million to 4 million, preferably from 1 million to 2 million.

6. The razor cartridge according to claim 1, whereim said lubricating material further comprises from 0.01 to 50% by weight, preferably from 2% to 40% by weight of said lubricating material of a copolymer of polyethylene oxide and polypropylene oxide, preferably a block copolymer, more preferably a tri-block copolymer.

7. The razor cartridge according to claim 6, wherein the copolymer of polyethylene oxide and polypropylene oxide has an average molecular weight of at least 5000, preferably in the range of from 10,000 to 20,000, more preferably from 11,000 to 15,000 and even more preferably from 12,000 to 13,000.

8. The razor cartridge according to claim 6, wherein the polyethylene oxide polymer is present at a level of from 70% to 90% by weight of said lubricating material.

9. The razor cartridge according to claim 1, wherein said container comprises four walls extending from said base, preferably enclosing said base.

10. The razor cartridge according to claim 8, wherein said skin contacting surface of said container further comprise a top, and wherein said top comprises at least one dispensing orifice.

11. The razor cartridge according to claim 8, wherein said skin contacting surface of said container comprises at least two dispensing orifices.

12. The razor cartridge according to claim 1, wherein the ratio of the cross sectional surface area of the skin contacting surface of the skin engaging member to the cross sectional surface area of said orifice is from 50:1 to 1:1, preferably from 25:1 to 2:1.

13. The razor cartridge according to claim 8, wherein said container is a least 60%, preferably at least 80% filled by volume with said lubricating material.

14. The razor cartridge according to claim 1, wherein said lubricating material is in the form of a compressed powder.

15. A razor cartridge according to claim 1, wherein said razor skin engaging member is located "aft" said at least two blades.

**Patentansprüche**

1. Rasierklingeneinheit, umfassend ein Gehäuse, umfassend

   a) zwei oder mehr Klingen, die eine Klingenrasierebene (P) bilden, und
   b) mindestens ein Hautberührungselement, wobei das Hautberührungselement Folgendes umfasst:

   i) einen Behälter mit einer Basis und mindestens einer Wand, die im Wesentlichen vertikal auf eine maximale Höhe (h) und vorzugsweise senkrecht zu der Klingenrasierebene (P) und einer Hautkontaktoberfläche, die vorzugsweise eine Abgabeöffnung aufweist, verläuft, und
   ii) ein Gleitmittelmaterial oder eine Mischung davon, die freisetzbar und im Wesentlichen innerhalb des Behälters enthalten ist, wobei das Gleitmittelmaterial von 50 Gew.-% bis 99 Gew.-% ein Polyethylenoxidpolymer oder eine Mischung davon umfasst, wobei das Polyethylenoxidpolymer ein durchschnittliches Molekulargewicht von 0,5 Millionen bis 10 Millionen aufweist, und wobei
   die Höhe (h) der Wand des Behälters von 0,1 mm über der Klingenebene (P) bis 0,6 mm unter der Klingenebene (P) ist, und so ausgewählt, dass die Beziehung zwischen dem Molekulargewicht des Polyethylenoxidpolymers und der maximalen Höhe der Wand durch den Bereich unter der Linie gemäß der folgenden Formel dargestellt wird:

$$\text{Molekulargewicht (Million) Polyethylenoxidpolymer} = 10 * h\ (mm) + 7.$$

2. Rasierklingeneinheit nach Anspruch 1, wobei das Molekulargewicht des Polyethylenoxidpolymers und die maximale Höhe (h) der mindestens einen Wand des Behälters so ausgewählt sind, dass die Beziehung zwischen dem Molekulargewicht des Polyethylenoxidpolymers und der maximalen Höhe der Wand durch den Bereich unter der Linie gemäß folgender Formel dargestellt wird:

$$\text{Molekulargewicht (Million) Polyethylenoxidpolymer} = 15 * h\ (mm) + 6.$$

3. Rasierklingeneinheit nach Anspruch 1, wobei die Höhe der Wand (h) des Behälters von 0 mm bis 0,3 mm unter der Klingenebene (P) ist.

4. Rasierklingeneinheit nach Anspruch 1, wobei das Polyethylenoxidpolymer ein durchschnittliches Molekulargewicht von 1 Million bis 6 Millionen, vorzugsweise von 1 Million bis 5 Millionen aufweist.

5. Rasierklingeneinheit nach Anspruch 1, wobei das Polyethylenoxidpolymer ein Molekulargewicht von 1 Million bis 4 Millionen, vorzugsweise von 1 Million bis 2 Millionen aufweist.

6. Rasierklingeneinheit nach Anspruch 1, wobei das Gleitmittelmaterial ferner von 0,01 bis 50 Gew.-%, vorzugsweise von 2 Gew.-% bis 40 Gew.-% des Gleitmittelmaterials ein Copolymer von Polyethylenoxid und Polypropylenoxid, vorzugsweise ein Blockcopolymer, mehr bevorzugt ein Triblockcopolymer, umfasst.

7. Rasierklingeneinheit nach Anspruch 6, wobei das Copolymer von Polyethylenoxid und Polypropylenoxid ein durchschnittliches Molekulargewicht von mindestens 5000, vorzugsweise im Bereich von 10.000 bis 20.000, mehr bevorzugt von 11.000 bis 15.000 und noch mehr bevorzugt von 12.000 bis 13.000 aufweist.

**8.** Rasierklingeneinheit nach Anspruch 6, wobei das Polyethylenoxidpolymer in einer Konzentration von 70 Gew.-% bis 90 Gew.-% des Gleitmittelmaterials vorliegt.

**9.** Rasierklingeneinheit nach Anspruch 1, wobei der Behälter vier Wände umfasst, die von der Basis ausgehen und die Basis vorzugsweise umschließen.

**10.** Rasierklingeneinheit nach Anspruch 8, wobei die Hautkontaktoberfläche des Behälters ferner eine Oberseite umfasst, und wobei die Oberseite mindestens eine Abgabeöffnung umfasst.

**11.** Rasierklingeneinheit nach Anspruch 8, wobei die Hautkontaktoberfläche des Behälters mindestens zwei Abgabeöffnungen umfasst.

**12.** Rasierklingeneinheit nach Anspruch 1, wobei das Verhältnis der Querschnittsfläche der Hautkontaktoberfläche des Hautberührungselements zur Querschnittsfläche der Öffnung von 50:1 bis 1:1, bevorzugt von 25:1 bis 2:1, beträgt.

**13.** Rasierklingeneinheit nach Anspruch 8, wobei der Behälter zu mindestens 60 Vol.-%, vorzugsweise mindestens 80 Vol.-% mit dem Gleitmittelmaterial gefüllt ist.

**14.** Rasierklingeneinheit nach Anspruch 1, wobei das Gleitmittelmaterial in der Form eines komprimierten Pulvers vorliegt.

**15.** Rasierklingeneinheit nach Anspruch 1, wobei das Rasierer-Hautberührungselement "nach" den mindestens zwei Klingen angeordnet ist.

**Revendications**

**1.** Cartouche de rasoir comprenant un logement comprenant

a) deux lames ou plus formant un plan de rasage de lame (P) et

b) au moins un élément de mise en prise de peau, ledit élément de mise en prise de peau comprenant :

i) un récipient ayant une base et au moins une paroi s'étendant de manière essentiellement verticale à une hauteur maximale (h) et de préférence perpendiculaire audit plan de rasage de lame (P) et une surface de contact de peau ayant de préférence un orifice de distribution, et

ii) un matériau lubrifiant ou un mélange de celui-ci contenu de manière libérable et essentiellement au sein dudit récipient, ledit matériau lubrifiant comprenant de 50 % à 99 % en poids d'un polymère d'oxyde de polyéthylène ou un mélange de celui-ci, dans laquelle ledit polymère d'oxyde de polyéthylène a une masse moléculaire moyenne allant de 0,5 million à 10 millions et dans laquelle

ladite taille (h) de ladite paroi dudit récipient va de 0,1 mm au-dessus dudit plan de lames (P) à 0,6 mm en dessous dudit plan de lames (P) et est choisie de telle sorte que la relation entre ladite masse moléculaire dudit polymère d'oxyde de polyéthylène et ladite hauteur maximale de ladite paroi est représentée par l'aire sous la ligne selon la formule suivante :

$$\text{masse moléculaire (million) polymère d'oxyde de polyéthylène} = 10 * h \text{ (mm)} + 7.$$

**2.** Cartouche de rasoir selon la revendication 1, dans laquelle ladite masse moléculaire dudit polymère d'oxyde de polyéthylène et ladite hauteur maximale (h) de ladite au moins une paroi dudit récipient sont choisies de telle sorte que la relation entre ladite masse moléculaire dudit polymère d'oxyde de polyéthylène et ladite hauteur maximale de ladite paroi est représentée par l'aire sous la ligne selon la formule :

$$\text{Masse moléculaire (million) polymère d'oxyde de polyéthylène} = 15 * h \text{ (mm)} + 6.$$

**3.** Cartouche de rasoir selon la revendication 1, dans laquelle ladite taille de ladite paroi (h) dudit récipient va de 0 mm à 0,3 mm en dessous dudit plan de lames (P).

**4.** Cartouche de rasoir selon la revendication 1, dans laquelle ledit polymère d'oxyde de polyéthylène a une masse moléculaire moyenne allant de 1 million à 6 millions, de préférence de 1 million à 5 millions.

**5.** Cartouche de rasoir selon la revendication 1, dans laquelle ledit polymère d'oxyde de polyéthylène a une masse moléculaire allant de 1 million à 4 millions, de préférence de 1 million à 2 millions.

**6.** Cartouche de rasoir selon la revendication 1, dans laquelle ledit matériau lubrifiant comprend en outre de 0,01 à 50 % en poids, de préférence de 2 % à 40 % en poids dudit matériau lubrifiant d'un copolymère d'oxyde de polyéthylène et d'oxyde de polypropylène, de préférence un copolymère séquencé, plus préférablement un copolymère tri-séquencé.

**7.** Cartouche de rasoir selon la revendication 6, dans laquelle le copolymère d'oxyde de polyéthylène et d'oxyde de polypropylène a une masse moléculaire moyenne d'au moins 5000, de préférence dans la plage allant de 10 000 à 20 000, plus préférablement de 11 000 à 15 000 et même plus préférablement de 12 000 à 13 000.

**8.** Cartouche de rasoir selon la revendication 6, dans laquelle le polymère d'oxyde de polyéthylène est présent à un taux allant de 70 % à 90 % en poids dudit matériau lubrifiant.

**9.** Cartouche de rasoir selon la revendication 1, dans laquelle ledit récipient comprend quatre parois s'étendant à partir de ladite base, enfermant de préférence ladite base.

**10.** Cartouche de rasoir selon la revendication 8, dans laquelle ladite surface de contact de peau dudit récipient comprend en outre un sommet, et dans laquelle ledit sommet comprend au moins un orifice de distribution.

**11.** Cartouche de rasoir selon la revendication 8, dans laquelle ladite surface de contact de peau dudit récipient comprend au moins deux orifices de distribution.

**12.** Cartouche de rasoir selon la revendication 1, dans laquelle le rapport de la superficie en coupe transversale de la surface de contact de peau de l'élément de mise en prise de peau à la superficie en coupe transversale dudit orifice va de 50:1 à 1:1, de préférence de 25:1 à 2:1.

**13.** Cartouche de rasoir selon la revendication 8, dans laquelle ledit récipient est rempli en volume à au moins 60 %, de préférence au moins 80 % avec ledit matériau lubrifiant.

**14.** Cartouche de rasoir selon la revendication 1, dans laquelle ledit matériau lubrifiant est sous la forme d'une poudre comprimée.

**15.** Cartouche de rasoir selon la revendication 1, dans laquelle ledit élément de mise en prise de peau du rasoir est situé « derrière » lesdites au moins deux lames.

# Fig. 1

Fig. 2

Fig. 3

Fig. 4

Fig. 5

Fig. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7121754 B
- US 6298558 B
- US 5711076 A
- US 5134775 A
- US 6301785 B
- US 20090223057 A
- US 20060225285 A
- US 20110099815 A
- WO 2011047221 A
- WO 2011047222 A
- WO 2011049892 A
- WO 2011050130 A
- US 20120030945 A
- US 6442839 B
- US 6302785 B
- US 2008060201 A
- US 7581318 B
- US 7168173 B
- US 7197825 B
- US 6449849 B
- US 6161288 A
- US 7607230 B
- US 7024776 B
- US 20080034590 A
- US 20090049695 A1
- US 7069658 B
- US 6944952 B
- US 6594904 B
- US 6182365 B
- US D424745 S
- US 6185822 B
- US 5113585 A